# EUROPEAN PATENT SPECIFICATION

(11) **EP 1 544 256 B1**
(45) Date of publication and mention of the grant of the patent: **20.10.2010**
(21) Application number: 04257867.4
(22) Date of filing: 16.12.2004
(51) Int. Cl.: C09C 1/36

(54) **Aluminium oxide coated titanium dioxide particles and methods of preparing the same in presence of a densifying agent**
Aluminiumoxidbeschichtete Titandioxidpartikel und Verfahren zu deren Herstellung unter Verwendung von verdichtenden Agentien
Particules d'oxyde de titane recouverts d'oxyde d'aluminium et leurs procédés de préparation utilisant un agent densifiant

(30) Priority: 16.12.2003 US 737357
(43) Date of publication of application: 22.06.2005
(73) Proprietor: E.I. DU PONT DE NEMOURS AND COMPANY, Wilmington, DE 19898 (US)
(72) Inventor: Frerichs, Scott Rickbeil, Smyrna, Delaware 19977 (US); Morrison, William Harvey Jr., Wilmington, Delaware 19810 (US)
(74) Representative: Towler, Philip Dean

(56) References cited:
- EP-A- 0 244 180
- WO-A-93/22386
- US-A- 4 199 370
- US-A- 5 451 390
- US-A1- 2003 089 278

## Description

### FIELD OF THE INVENTION

The present invention relates to nanoparticle titanium dioxide particles and processes for preparing them. More specifically, the invention relates to nanoparticle titanium dioxide particles which are alumina treated in the presence of citric acid. Yet more specifically, the invention relates to nanoparticle titanium dioxide particles which are silica and alumina treated in the presence of citric acid. In addition, the invention relates to compositions comprising the particles of the invention.

### BACKGROUND OF THE INVENTION

The scientific and technological advantages of nanostructured particles and materials have been attracting considerable attention. The small size of nanoparticles (generally used to indicate particles less than 100 nm in diameter), which can be responsible for different useful properties (electronic, optical, electrical, magnetic, chemical, and mechanical), makes them suitable for a wide variety of industrial applications.

Titanium dioxide (TiO₂) nanoparticles are substantially transparent to visible light but can absorb and scatter ultraviolet light. Titanium dioxide has low toxicity and is non-irritating to the skin. TiO₂ nanoparticles are especially advantageous when added to products in which transparency to visible light is important but exposure to the degrading and harmful effects of ultraviolet light is a problem. Such applications include, without limit, cosmetics, sunscreens, protective coatings, such as clear coatings for exterior wood and automobiles, and plastics.

Titanium dioxide itself is known to be photoactive. Free radicals form on the surface of the titanium dioxide particle under the action of ultraviolet rays. While the photoactivity of titanium dioxide is beneficial for use of titanium dioxide in photo catalyzed reactions, in other uses the free radicals can lead to degradation reactions and yellowing which can be disadvantageous. Such other uses include, without limit, cosmetics, sunscreens and plastics, wood and automotive coatings, etc. Thus, there is a desire for techniques that can photo-passivate the titanium dioxide; that is, render the titanium dioxide more photostable.

Untreated titanium dioxide nanoparticles are known to be chemically reactive. Untreated titanium dioxide will form highly colored complexes with certain antioxidants, such as ascorbic acid and ascorbic acid 6-palmitate. These colored complexes limit the use of titanium dioxide nanoparticles in applications where white creams and lotions are desired, such as cosmetics and sunscreens. Effective methods for passivation of the chemical reactivity of titanium dioxide nanoparticles are therefore desired. Thus, there is a desire for techniques that can make titanium dioxide nanoparticles nonreactive to such antioxidants.

Titanium dioxide nanoparticles are often prepared and/or used as a dispersion of the particles in a fluid medium, where the dispersion is, for example, an emulsion, slurry, cream, lotion or gel. However, dry titanium dioxide nanoparticles can form agglomerates and be difficult to disperse. Consequently, there is a need for titanium dioxide nanoparticles that are photopassived, have a reduced tendency to form agglomerates, and are easy to disperse in a fluid medium.

### BRIEF SUMMARY OF THE INVENTION

The present invention relates to a process for treating titanium dioxide nanoparticles comprising
(a) forming a slurry of titanium dioxide nanoparticles;
(b) contacting the slurry of titanium dioxide nanoparticles with a densifying agent;
(c) contacting the slurry with a source of metal oxide selected from the group consisting of a source of alumina, a source of silica or both; and
(d) recovering the treated titanium dioxide nanoparticles formed in step (c).
In another embodiment, the invention relates to a process for treating titanium dioxide nanoparticles comprising
(a) forming a slurry of titanium dioxide nanoparticles;
(b) contacting the slurry of titanium dioxide nanoparticles with a densifying agent;
(c) treating the slurry of step (b) with a source of silica under conditions sufficient to deposit silica onto the titanium dioxide nanoparticles in an amount ranging from about 5 weight percent to about 18 weight percent based on the weight of the titanium dioxide nanoparticles in the mixture;
(d) treating the slurry of step (c) with a source of alumina under conditions sufficient to deposit alumina in an amount ranging from about 5 weight percent to about 15 weight percent based on the weight of the titanium dioxide nanoparticles; and
(e) recovering the treated titanium dioxide nanoparticles formed in step (d).

The process of the instant invention has been found to produce titanium dioxide nanoparticles which are passivated as indicated by a high photo stability and/or high chemical stability. In addition the nanoparticles have a reduced tendency to form agglomerates.

The treated titanium dioxide nanoparticles of this invention can be used in sunscreen formulations and in thermoplastic compositions.

### DETAILED DESCRIPTION OF THE INVENTION

In the process of this invention, at least one of a source of silica and alumina can be added to a slurry of titanium dioxide nanoparticles, water and a densifying agent to form the treated titanium dioxide nanoparticles.

The present invention provides titanium dioxide nanoparticles which are treated, preferably surface treated, with amorphous alumina in the presence of a densifying agent. More specifically, the particles are coated in a wet treatment process with amorphous alumina in the presence of a densifying agent. Optionally, the particles are further treated, preferably surface treated, with amorphous silica also in the presence of a densifying agent.

The present invention also can provide titanium dioxide nanoparticles which are treated, preferably surface treated, with amorphous silica in the presence of a densifying agent. More specifically, the particles are coated in a wet treatment process with amorphous silica in the presence of a densifying agent. Optionally, the particles are further treated with amorphous alumina also in the presence of a densifying agent.

In one embodiment of this invention, a slurry of titanium dioxide nanoparticles is heated and a densifying agent is added to the slurry. The slurry is an aqueous mixture of the titanium dioxide particles, which are water insoluble. The slurry is pH adjusted to form a basic composition and then treated with a source of alumina or silica or both, typically sodium aluminate or sodium silicate. After treatment with the source of alumina or silica or both the slurry is held at a certain pH and elevated temperature for a period of time sufficient to cure the particles. An objective of the curing step is to deposit alumina and/or silica onto the particles, more typically, by coating the particles with a layer of alumina and/or silica.

In one embodiment of the invention the initial temperature of the slurry is optimally greater than about 30°C, typically greater than about 35 °C, even more typically greater than about 50 °C, and yet more typically above about 60 °C. Temperatures can range from about 30 to about 100°C, more typically in the range of about 40°C to about 100°C and still more typically from about 60° to about 100°C, although lower temperatures might also be effective. In one embodiment of the invention the initial temperature of the slurry is optimally greater than about 50 °C, typically above about 60 °C, more typically in the range of about 60° to about 100°C, although lower temperatures might also be effective. The amount of the source of alumina and/or silica is optimally in the range of between about 5 and about 15% as Al₂O₃ based on weight of untreated TiO₂.

A strong mineral acid can be employed during the alumina and/or silica treatment. Any strong mineral acid, including but not limited to HCI, HNO₃, and H₂SO₄ could be used. The optimal acid addition time for a small lab scale batch process ranges from 0.5 to about 2.0 minutes per 1 % Al₂O₃ and/or SiO₂ added (up to 30 minutes per 1 % Al₂O₃ and/or SiO₂ for large plant scale batches). Longer times can lead to better product but at the expense of rate.

After adding the alumina and/or silica, the pH of the slurry is typically held at a neutral level, optimally at 7 ± 0.5. Higher values might lead to undesired phases, particularly for alumina; lower values to incomplete deposition.

The alumina and/or silica treated slurry is then held for a period of time sufficient to deposit alumina and/or silica onto the titanium dioxide particles typically by forming a coating of alumina and/or silica on the titanium dioxide particles. The holding time is typically 3 minutes per 1% alumina and/or silica for small lab scale batches (up to 20 minutes per 1% alumina and/or silica for large plant batches). Shorter times can be used but the treatment may not be as effective. This holding step is typically carried out while maintaining a neutral pH and elevated temperature. Thus the pH usually is maintained at 7.0 ± 0.5. In one embodiment of the invention the temperature of the slurry is optimally greater than about 30°C, typically greater than about 35 °C, even more typically greater than about 50 °C, and yet more typically above about 60 °C. Temperatures can range from about 30 to about 100°C, more typically in the range of about 40°C to about 100°C and still more typically from about 60° to about 100°C, although lower temperatures might also be effective. The temperature is usually maintained at about 50°C, typically above about 45°C, more typically at about 55 to about 60°C.

Particulate compositions of the present invention generally include from about 3 to about 20%, more typically from about 5 to about 15% amorphous alumina based on the weight of the untreated TiO₂. Particulate compositions of the present invention generally can include from about 2 to about 20, generally from about 5 to about 18 % amorphous silica based on the weight of the untreated TiO₂

The alumina and/or silica treated titanium dioxide nanoparticles, usually, are then filtered, washed and dried. The final particles are in a size range less than pigmentary; typically the average particle size in diameter is between about 80 and about 125 nanometers, sometimes less than about 100 nanometers determined by techniques well known in the art such as scanning electron micrograph.

In a preferred embodiment of this invention the slurry is treated with both a source of silica and a source of alumina. In this embodiment, a slurry of titanium dioxide nanoparticles is heated and densifying agent is added to the slurry. The slurry is an aqueous mixture of the titanium dioxide particles, which are water insoluble. The slurry is then pH adjusted to form a basic composition and then treated with a source of silica, typically sodium silicate. The pH is decreased to a more neutral level by addition of acid, after which the slurry is treated with a source of alumina, typically sodium aluminate. After treatment with the source of silica and alumina the slurry is held at a certain pH and elevated temperature for a period of time sufficient to cure the particles. An objective of the curing step is to deposit silica and alumina onto the particles, more specifically, by coating the particles with a layer of silica and a layer of alumina.

The treatment occurs in the presence of a densifying agent. The densifying agent is important for densifying the coatings of silica and/or alumina. Suitable densifying agents include citric acid or a source of phosphate ion such as phosphoric acid or a source of sulfate ion such as sodium sulfate. Citric acid is the preferred densifying agent because of its dispersion enhancing properties. A useful amount of densifying agent is an amount sufficient to adequately densify the silica and alumina coatings. An excess of densification agent will maximize densification of the silica and alum ina coatings but may lead to waste of the densifying agent. Suitable amounts of the densifying agent can be in the range of about 0.5% to about 3.0%, more typically from about 0.8% to about 2.4% based on weight of untreated TiO₂.

The concentration of TiO₂ in the slurry ranges from about 50g/ml to about 500 g/l more typically from about 125 to 250 grams per liter, although lower levels are also possible. Good coating consistency has been found with a relatively low concentration slurry. The temperature of the slurry usually ranges from about 30 to about 100°C, typically about 35 to about 100°C, more typically about 45 to about 100°C, optimally from about 85 to about 100 °C, although lower or higher temperatures might also be effective.

Before adding the source of silica, the slurry is maintained in the alkaline range, typically the pH is above 8.5, more typically 9.0 or higher although this may depend on the equipment used (lower pH may be possible for continuous wet treatment). The optimal silica deposition weight is typically between about 2 and about 20, more typically from about 5 to about 18% as SiO₂ based on weight of untreated TiO₂. However, improvements are likely to be seen at any silica level.

Any strong mineral acid, including HCI, HNO₃ and H₂SO₄ may be used to neutralize the slurry prior to alumina treatment. The optimal acid addition time for batch process ranges from 0.5 to about 4 minutes per 1% SiO₂ added for small lab scale batches (up to 30 minutes per 1 % SiO₂ for large plant scale batches). Longer times can lead to better product at the expense of rate.

The silica treated slurry is then held for a period of time which is preferably sufficient to deposit a coating of silica on the titanium dioxide particles. The holding time is typically 5 minutes per 1% silica for small lab scale batches (up to 20 minutes per 1 % silica for large plant scale batches). Shorter times can be used but the coating may not be as effective. This holding step is typically carried out while maintaining a neutral to alkaline pH and elevated temperature. Thus, the pH usually is maintained at 7.0 ± 1.0 and higher, typically up to and including about 10. The temperature is usually maintained above about 80 °C, typically above about 90°C, more typically at about 95 to about 100°C.

In the alumina treatment the initial temperature of the slurry is optimally greater than about 80 °C, typically above about 90 °C, more typically in the range of about 95° to about 100°C, although lower temperatures might also be effective (or even more effective but at the expense of energy and time necessary to chill the slurry). Aluminate amount is optimally in the range of between about 5 and about 15% as Al₂O₃ based on weight of untreated TiO₂.

Any strong mineral acid can be employed during the alumina treatment including HCI, HNO₃, and H₂SO₄. The optimal acid addition time for a small lab scale batch process ranges from 0.5 to about 2.0 minutes per 1 % Al₂O₃ added (up to 30 minutes per 1 % Al₂O₃ for large plant scale batches). Longer times can lead to better product at the expense of rate.

After adding the alumina, the pH of the slurry is typically held at a neutral level, optimally at 7 ± 0.5. Higher values might lead to undesired alumina phase; lower values to incomplete deposition.

The alumina treated slurry is then held for a period of time sufficient to form a coating of alumina on the titanium dioxide particles to which a silica coating has been deposited. The holding time is typically 3 minutes per 1% alumina for small lab scale batches (up to 20 minutes per 1% alumina for large plant batches). Shorter times can be used but the coating may not be as effective. This holding step is typically carried out while maintaining a neutral pH and elevated temperature. Thus the pH usually is maintained at 7.0 ± 0.5. The temperature is usually maintained at about 50°C, typically above about 45°C, more typically at about 55 to about 60°C.

Silica and alumina treated particulate compositions of the present invention generally can include from about 2 to about 20, generally from about 5 to about 18 % amorphous silica based on the weight of the untreated TiO₂ and from about 3 to about 20%, more typically from about 5 to about 15% amorphous alumina based on the weight of the untreated TiO₂.

The silica and alumina treated titanium dioxide nanoparticles, usually, are then filtered, washed and dried. The final particles are in a size range less than pigmentary; typically the average particle size in diameter is between about 80 and about 125 nanometers, additionally less than about 100 nanometers.

Any titanium dioxide nanoparticles can be suitable as a starting material for the process of this invention. As an example, titanium dioxide nanoparticles suitable as the starting material are described in U.S. Patent Nos. 5,451,390; 5,672,330; and 5,762,914. Titanium dioxide P25 is an example of a suitable commercial product available from Degussa. Other commercial suppliers of titanium dioxide nanoparticles include Kemira, Sachtleben and Tayca.

The titanium dioxide nanoparticle starting materials typically have an average particle size diameter of less than 100 nanometers (nm) as determined by dynamic light scattering which measures the particle size distribution of particles in liquid suspension. The particles are typically agglomerates which may range from about 3 nm to about 6000 nm. Any process known in the art can be used to prepare such particles. The process may involve vapor phase oxidation of titanium halides or solution precipitation from soluble titanium complexes, provided that titanium dioxide nanoparticles are produced.

A preferred process to prepare titanium dioxide nanoparticle starting material is by injecting oxygen and titanium halide, preferably titanium tetrachloride, into a high-temperature reaction zone, typically ranging from 400 to 2000 degrees centrigrade. Under the high temperature conditions present in the reaction zone, nanoparticles of titanium dioxide are formed having high surface area and a narrow size distribution. The energy source in the reactor may be any heating source such as a plasma torch. Optionally, the reactor may also include a flow homogenizer that ensures that feeds from the reactant inlets enter the reactor chamber downstream of the recirculation zone induced by the high temperature gas discharge. A flow homogenizer is described in U.S. Provisional Patent Application No. 60/434158 filed on December 17, 2002.

The titanium dioxide starting material can be substantially pure titanium dioxide or may contain other inorganic material such as metal oxides. Examples include one or more of silica, alumina, zirconia and magnesia which can be incorporated into the particle using techniques known by those skilled in the art, for example these metal oxides can be incorporated when the titanium compounds are co-oxidized or coprecipitated with other metal oxide compounds. If such co-metals are present, they are preferably present in an amount of about 0.1 to about 5 % based on the total metal oxide weight. The titanium dioxide starting material may also have one or more such metal oxide coatings applied using techniques known by those skilled in the art prior to treatment in accordance with this invention. In one embodiment of the invention, a slurry of substantially pure titanium dioxide is "pretreated" with alumina prior to contacting the slurry with citric acid. The pretreatment is typically to an amount of about 1 to about 4% based on the total metal oxide weight.

Typically, for alumina pretreated titanium dioxide, the final alumina level of products made by the invention is about 2.5% higher if the TiO₂ is pretreated with alumina.

Benefits have been found when the titanium dioxide nanoparticle starting material contains alumina, in a coating or by incorporation into the particle. For example, it has been found that the silica treatment step is more effective when applied to titanium dioxide particles that contain alumina. In addition, it has been found that the chemical stability (determined by the Vitamin C Yellowing Test which is described below) is higher and fewer oversized particles are produced by the process, specifically about 10% fewer oversized particles, as compared to a titanium dioxide starting material that does not contain alumina. By the term "oversized particles" it is meant agglomerates which are greater in diameter than about 200 nm, as determined by the MICROTRAC ultrafine particle analyzer.

The titanium dioxide starting material can also have an organic coating which may be applied using techniques known by those skilled in the art. A wide variety of organic coatings are known. Organic coatings employed for pigment-sized titanium dioxide may be utilized to coat nanoparticles. Examples of organic coatings that are well known to those skilled in the art include fatty acids, such as stearic acid; fatty acid esters; fatty alcohols, such as stearyl alcohol; polyols such as trimethylpropane diol or trimethyl pentane diol; acrylic monomers, oligomers and polymers; and silicones, such as polydimethylsiloxane and reactive silicones such as methylhydroxysiloxane.

Organic coating agents can include but are not limited to carboxylic acids such as adipic acid, terephthalic acid, lauric acid, myristic acid, palmitic acid, stearic acid, oleic acid, salicylic acid, malic acid, maleic acid, and esters, fatty acid esters, fatty alcohols, such as stearyl alcohol, or salts thereof, polyols such as trimethylpropane diol or trimethyl pentane diol; acrylic monomers, oligomers and polymers. In addition, silicon-containing compounds are also of utility. Examples of silicon compounds include but are not limited to a silicate or organic silane or siloxane including silicate, organoalkoxysilane, aminosilane, epoxysilane, and mercaptosilane such as hexyltrimethoxysilane, octyltriethoxysilane, nonyltriethoxysilane, decyltriethoxysilane, dodecyltriethoxysilane, tridecyltriethoxysilane, tetradecyltriethoxysilane, pentadecyltriethoxysilane, hexadecyltriethoxysilane, heptadecyltriethoxysilane, octadecyltriethoxysilane, N-(2-aminoethyl) 3-aminopropylmethyl dimethoxysilane, N-(2-aminoethyl) 3-aminopropyl trimethoxysilane, 3-aminopropyl triethoxysilane, 3-glycidoxypropyl trimethoxysilane, 3-glycidoxypropyl methyldimethoxysilane, 3-mercaptopropyl trimethoxysilane and combinations of two or more thereof. Polydimethylsiloxane and reactive silicones such as methylhydroxysiloxane may also be useful.

The particles may also be coated with a silane having the formula:

RₓSi(R')₄₋ₓ

wherein
R is a nonhydrolyzable aliphatic, cycloaliphatic or aromatic group having at least 1 to about 20 carbon atoms;
R' is a hydrolyzable group such as an alkoxy, halogen, acetoxy or hydroxy or mixtures thereof; and
x=1 to 3.
For example, silanes useful in carrying out the invention include hexyltrimethoxysilane, octyltriethoxysilane, nonyltriethoxysilane, decyltriethoxysilane, dodecyltriethoxysilane, tridecyltriethoxysilane, tetradecyltriethoxysilane, pentadecyltriethoxysilane, hexadecyltriethoxysilane, heptadecyltriethoxysilane and octadecyltriethoxysilane. Additional examples of silanes include, R=8-18 carbon atoms; R'=chloro, methoxy, hydroxy or mixtures thereof; and x=1 to 3. Preferred silanes are R=8-18 carbon atoms; R'=ethoxy; and x=1 to 3. Mixtures of silanes are contemplated equivalents. The weight content of the treating agent, based on total treated particles can range from about 0.1 to about 10 wt. %, additionally about 0.7 to about 7.0 wt. % and additionally from about 0.5 to about 5 wt %.

The titanium dioxide particles of this invention can be silanized as described in U.S. Patent Nos. 5,889,090; 5,607,994; 5,631,310; and 5,959,004.

The titanium dioxide starting material and/or the final silica and alumina treated titanium dioxide particles of this invention may be treated to have any one or more of the foregoing organic coatings.

Titanium dioxide nanoparticles made according to the present invention may be used with advantage in various applications including sunscreens and cosmetic formulations; coatings formulations including automotive coatings, wood coatings, and surface coatings; chemical mechanical planarization products; catalyst products; photovoltaic cells; plastic parts, films, and resin systems including agricultural films, food packaging films, molded automotive plastic parts, and engineering polymer resins; rubber based products including silicone rubbers; textile fibers, woven and nonwoven applications including polyamide, polyaramid, and polyimides fibers products and nonwoven sheets products; ceramics; glass products including architectural glass, automotive safety glass, and industrial glass; electronic components; and other uses in which photo and chemically passivated titanium dioxide nanoparticles will be useful.

One area of increasing demand for titanium dioxide nanoparticles is in cosmetic formulations, particularly in sunscreens as a sunscreen agent. Titanium dioxide nanoparticles provide protection from the harmful ultraviolet rays of the sun (UV A and UV B radiation). Both UV A and UV B radiation have been implicated in numerous skin problems, ranging from causing freckles, sunburn (erythema), and wrinkles, and premature aging. In addition, UV A radiation has been linked with skin cancer.

A dispersant is usually required to effectively disperse titanium dioxide nanoparticles in a fluid medium. Careful selection of dispersants is important. Typical dispersants for use with titanium dioxide nanoparticles include aliphatic alcohols, saturated fatty acids and fatty acid amines.

The titanium dioxide nanoparticles of this invention can be incorporated into a sunscreen formulation. Typically the amount of titanium dioxide nanoparticles can be up to about 25 wt.%, typically from about 0.1 wt.% to up to 15 wt. %, even more preferably up to 6 wt.%, based on the weight of the formulation, the amount depending upon the desired sun protection factor (SPF) of the formulation. The sunscreen formulations are usually an emulsion and the oil phase of the emulsion typically contains the UV active ingredients such as the titanium dioxide particles of this invention. Sunscreen formulations typically contain in addition to water, emollients, humectants, thickeners, UV actives, chelating agents, emulsifiers, suspending agents (typically if using particulate UV actives), waterproofers, film forming agents and preservatives.

Specific examples of preservatives include parabens. Specific examples of emollients include octyl palmitate, cetearyl alcohol, and dimethicone. Specific examples of humectants include propylene glycol, glycerin, and butylene glycol. Specific examples of thickeners include xanthan gum, magnesium aluminum silicate, cellulose gum, and hydrogenated castor oil. Specific examples of chelating agents include disodium ethylene diaminetetraacetic acid (EDTA) and tetrasodium EDTA. Specific examples of UV actives include ethylhexyl methoxycinnamate, octocrylene, and titanium dioxide. Specific examples of emulsifiers include glyceryl stearate, polyethyleneglycol-100 stearate, and ceteareth-20. Specific examples of suspending agents include diethanolamine-oleth-3-phosphate and neopentyl glycol dioctanoate. Specific examples of waterproofers include C30-38 olefin/isopropyl maleate/MA copolymer. Specific examples of film forming agents include hydroxyethyl cellulose and sodium carbomer. Numerous means are available for preparing dispersions of titanium dioxide nanoparticles containing dispersants. Intense mixing, such as milling and grinding may be needed, for example, to break down agglomerates into smaller particles. To facilitate use by the customer, producers of titanium dioxide nanoparticles may prepare and provide dispersions of the particles in a fluid medium which are easier to incorporate into formulations.

Because of the reduced photo activity of the titanium dioxide particles of this invention, they can be beneficial in products which degrade upon exposure to UV light energy.

Thus in one embodiment, the invention is directed to a coating composition suitable for protection against ultraviolet light comprising an additive amount suitable for imparting protection against ultraviolet light of the silica and alumina coated titanium dioxide nanoparticles made in accordance with this invention dispersed in a protective coating formulation.

Water based wood coatings, especially colored transparent and clear coatings benefit from a UV stabilizer which protects the wood. Organic UV absorbers are typically hydroxybenzophenones and hydroxyphenyl benzotriazoles. A commercially available UV absorber is sold under the trade name Tinuvin^{TM} by Ciba. These organic materials, however, have a short life and decompose on exterior exposure. Replacing the organic material with titanium dioxide nanoparticles would allow very long lasting UV protection. The titanium dioxide passivated in accordance with this invention prevents the titanium dioxide from oxidizing the polymer in the wood coating, and is sufficiently transparent so the desired wood color can be seen. Because most wood coatings are water based, the titanium dioxide needs to be dispersible in the water phase. Various organic surfactants known in the art can be used to disperse the titanium dioxide nanoparticles in water.

Many cars are now coated with a clear layer of polymer coating to protect the underlying color coat, and ultimately the metal body parts. This layer has organic UV protectors, and like wood coatings, a more permanent replacement for these materials is desired. The titanium dioxide nanoparticles made in accordance with this invention are sufficiently transparent, and passivated for this application. The clear coat layers are normally solvent based, but can also be water based. Such coatings are well known in the art. The titanium dioxide nanoparticles can be modified for either solvent or water based systems with appropriate surfactants or organic surface treatments.

Titanium dioxide nanoparticles can be used to increase the mechanical strength of thermoplastic composites. Most of these applications also require a high degree of transparency and passivation so underlying color or patterns are visible and the plastic is not degraded by the photoactivity of the titanium dioxide nanoparticles. The titanium dioxide nanoparticles must be compatible with the plastic and easily compounded into it. This application typically employs organic surface modification of the titanium dioxide nanoparticles as described herein above. The foregoing thermoplastic composites are well known in the art.

Polymers which are suitable as thermoplastic materials for use in the present invention include, by way of example but not limited thereto, polymers of ethylenically unsaturated monomers including olefins such as polyethylene, polypropylene, polybutylene, and copolymers of ethylene with higher olefins such as alpha olefins containing 4 to 10 carbon atoms or vinyl acetate, etc.; vinyls such as polyvinyl chloride, polyvinyl esters such as polyvinyl acetate, polystyrene, acrylic homopolymers and copolymers; phenolics; alkyds; amino resins; epoxy resins, polyamides, polyurethanes; phenoxy resins, polysulfones; polycarbonates; polyether and chlorinated polyesters; polyethers; acetal resins; polyimides; and polyoxyethylenes. The polymers according to the present invention also include various rubbers and/or elastomers either natural or synthetic polymers based on copolymerization, grafting, or physical blending of various diene monomers with the above-mentioned polymers, all as generally well known in the art. Thus generally, the present invention is useful for any plastic or elastomeric compositions ( which can also be pigmented with pigmentary TiO₂). For example, but not by way of limitation, the invention is felt to be particularly useful for polyolefins such as polyethylene and polypropylene, polyvinyl chloride, polyamides and polyester.

In one embodiment, the invention herein can be construed as excluding any element or process step that does not materially affect the basic and novel characteristics of the composition or process. Additionally, the invention can be construed as excluding any element or process step not specified herein.

### TEST METHODS

### Vitamin C Yellowing Test For Chemical Stability:

A standard solution of 6.25% ascorbic acid palmitate (L-ascorbic acid 6-palmitate, 99%, CAS #137-66-6, available commercially from Alfa Aesar) in octyl palmitate (hexadecanoic acid 2-ethylhexyl ester, CAS #29806-73-3, available under the name "Ceraphyl" by VanDyk) is prepared. Using a spatula and glass plate or Hoover Muller Model M5, 1.9 +0.05 ml of the solution is thoroughly mixed with 0.4 +0.01g sample of titanium dioxide to be tested. The mixture is drawn down onto a white lacquered 3" x 5" card using a 6 mil Bird film applicator to form the test film. The color (L*a*b*) of the test film is measured using a hand-held spectrocolorimeter, such as Byk-Gardner Model CB-6805 which is warmed-up prior to taking the color reading, calibrated and set up to use D65/10 degree (illuminant/observer). In the same manner as the test film, a blank film is prepared using neat octyl palmitate and ultrafine titanium dioxide. The color of the blank film is measured in the same way as the color of the test film. The delta b* value is determined by comparing the color of the test and blank films. The delta b* value is a measure of chemical activity.

### UPA Particle Size Distribution

The MICROTRAC ULTRAFINE PARTICLE ANALYZER (UPA) (a trademark of Leeds and Northrup, North Wales, PA) uses the principle of dynamic light scattering to measure the particle size distribution of particles in liquid suspension. Leeds and Northrup, North Wales, PA manufacture the instrument. The measured size range is 0.003µm to 6µm (3nm to 6000nm). Use 2.55 for the refractive index of TiO₂ when setting up the UPA analysis. The dry particle sample needs to be prepared into a liquid dispersion to carry out the measurement. An example procedure is as follow:
(1) Weigh out 0.08g dry powder.
(2) Add 79.92g 0.1 % tetra sodium pyrophosphate (TSPP) solution in water to make a 0.1 wt.% suspension.
(3) Sonify the suspension for 10 minutes using an ultrasonic probe. The suspension should be cooled in a water-jacketed beaker during sonication.
(4) When sonication is complete, draw an aliquot for analysis. Note, hydrophobic particles must first be wetted with a few drops of ethanol before adding into solution of TSPP.

The results of these tests were reported below for each of the examples.

### EXAMPLES

### Example 1

In a half gallon plastic jug containing 100 g nanometric titanium dioxide made by RF plasma oxidation according to US 2002/0155059A1 800 mls total volume deionized polished water was added and the mixture was stirred. The nanometric titanium dioxide starting material had a mean particle size of 90 nm, 10 wt% of particles less than 50 nm in size, and 90% of particles less than 150 nm in size as measured by the Microtrac UPA dynamic light scattering instrument. The mixture was sonicated for 10 minutes at a power of 7 and screened through a 325 mesh sieve. The screened mixture was added to a 2000 ml stainless steel beaker equipped with an electric stirrer, temperature probe and pH probe. The mixture was rapidly stirred using a propeller blade.

The initial pH was 1. The mixture was heated to 60°C and the pH was adjusted to 7.1 with 50% NaOH solution (8.2g). Then 9.0g sodium aluminate (27.8 wt% alumina) was added. The pH was 10.8. The mixture was stirred for 15 minutes.

The mixture was heated to 92°C. The pH was 10.0. Then 1.6 g 50% citric acid solution was added. The pH after citric acid addition was 8.8. The pH was adjusted to 10.7 with 50% NaOH solution. Then 21.5 g sodium silicate (27 wt% silica) was added with strong stirring. The pH was 10.7. Over about 15 minutes concentrated (38%) hydrochloric acid solution was added to reduce the pH to 7 (17.7g HCI). The mixture was stirred for 45 minutes at 92-95°C. The heat was stopped and the pH was reduced to the range of 6-8 with concentrated (38% HCI) (13.5g) while adding 18.0 g sodium aluminate drop-wise over 15 minutes. The mixture was stirred for 20 minutes while maintaining a pH of 7. At the end of 20 minutes the temperature was 60. The pH was adjusted to 6.0 +0.3 with concentrated (38%) HCI. The mixture was stirred again for 5 minutes. The final mixture was filtered, washed with deionized polished water to <143 mhos/cm conductance (~3 liters water, 106 micro mhos/cm). The mixture was vacuum dried for about 30 minutes to form a cake then ethanol was added to cover the cake for about 15 minutes. The cake was then vacuum dried again for about 30 minutes. The cake was dried in a 125°C oven on a tray overnight. The dry particles were ground and sieved through a 35 mesh screen and dried again.
Measured SiO₂: 3.9%
Measured Al₂O₃: 5.7%

### Example 2

The following materials were added to a 1000 ml plastic beaker: 50.00 g Degussa P25 titanium dioxide and 400 ml deionized polished water. The mixture was stirred then sonicated for 3 minutes at a power of 7. The mixture was then poured into a 600 ml stainless steel beaker equipped with an electric stirrer, temperature probe and pH probe. The mixture was agitated using a propeller blade. The initial pH of the mixture was 3.3. The mixture was heated to about 95°C and 0.8 g citric acid 50% solution was added. The pH was 2.7. The pH was adjusted with 10% NaOH to a range of 9-9.5 by adding 3.8 g 50% NaOH solution. The neutral pH was maintained by adding 8.1 g concentrated (38%) HCI while adding 10.75 g sodium silicate drop wise over 14 minutes. The mixture was heated at 95°C for one hour at pH 9.5 with stirring at about 2600 rpm. The pH was lowered to 7 by adding 8.1 g concentrated (38%) HCI while 9 g sodium aluminate was added drop wise over 10 minutes. The heat was turned off and the mixture was stirred for 20 minutes at pH of 7. The temperature after 20 minutes was 75.5°C. The pH was adjusted to 6.0 +0.3 with HCI and stirred for 5 minutes.

The mixture was filtered, washed and dried and the dry particles were formed as in Example 1.
Measured SiO₂: 4.4%
Measured Al₂O₃: 3.2%

### Example 3

The treatment was performed as in Example 1 except no sodium aluminate was added prior to the addition of sodium silicate.
Measured SiO₂: 4.1 %
Measured Al₂O₃: 4.4%

### Example 4

The aqueous mixture of titanium dioxide was prepared, stirred then solicated and pH adjusted as in Example 1.The initial pH was 1.5. The mixture was heated to 60°C and the pH was adjusted to 7.3 with 50% NaOH solution (8.2g). Then 9.0g sodium aluminate (27.8 wt% alumina) was added. The pH was11.4. The mixture was stirred for 15 minutes.

The mixture was heated to 92°C. The pH was10.9. Then 4.8 g 50% citric acid solution was added. The pH after citric acid addition was 9.7. The pH was adjusted to 10.9 with 50% NaOH solution. Then 64.5 g sodium silicate (27 wt% silica) was added with strong stirring. The pH was 11.0. Over about 15 minutes concentrated (38%) hydrochloric acid solution was added to reduce the pH to 7 (23.5g.HCl). The mixture was stirred for 45 minutes at 2-95°C. The heat was stopped and the pH was reduced to the range of 6-8 with concentrated (38% HCI)(37.4 g) while adding 54.0 g sodium aluminate drop-wise over 13 minutes. The mixture was stirred for 20 minutes while maintaining a pH of 7. At the end of 20 minutes the temperature was 44°C. The pH was adjusted to 6.0 ±0.3 with concentrated (38%) HCI The mixture was stirred again for 5 minutes. The final mixture was filtered, washed with deionized polished water to <143 mhos/cm conductance (~3 liters water, 100 micro mhos/cm). The mixture was vacuum dried for about 30 minutes to form a cake then ethanol was added to cover the cake for about 15 minutes. The cake was then vacuum dried again for about 30 minutes. The cake was dried in a 125°C oven on a tray overnight. The dry particles were ground and sieved through a 35 mesh screen and dried again.
Measured SiO₂: 10.1%
Measured Al₂O₃:14.5%

### Example 5

In this Example, no citric acid was used. The aqueous mixture of titanium dioxide was prepared, stirred, sonicated and pH adjusted as in Example 1. It was then heated to 60 °C and stirred for 15 minutes, then filtered, washed, and dried as in Example 1.
Measured SiO₂: 0.0%
Measured Al₂O₃: 0.0%

### Example 6

In this Example, no citric acid was used. The following materials were added to a 1000 ml plastic beaker: 50.00 g Degussa P25 titanium dioxide and 400 ml deionized polished water. The mixture was stirred then sonicated for 3 minutes at a power of 7. The mixture was then agitated with an electric stirrer motor and heated to 92°C. The initial pH was 3.2. The pH was adjusted to 9.2 using 1.4 g 10% NaOH. The pH of the mixture was maintained in a range of 9-10 using HCI (18%, 10.3 g, 50% dilute) while 18.5 g sodium silicate solution (27 wt.% SiO₂) was added drop wise over 8 minutes. The mixture was heated for one hour.

The mixture was filtered, washed and dried as described in Example 1 and the particles were ground and sieved through a mesh screen and dried again.
Measured SiO₂: 8.33%

### Example 7

In this Example, no citric acid was used. The aqueous mixture of titanium dioxide was prepared, stirred then sonicated as described in Example 2. The initial pH was in the range of 3.3-3.6. The mixture was heated to about 91 °C. The pH was adjusted to 9.4 using 1.24 g 10% NaOH. The pH of the mixture was maintained in a range of 9-9.5 using HCI (18%, 20.63 g, 50% dilute) while adding 37.04 g sodium silicate solution (27 wt.% SiO₂) drop wise over about 40 minutes. The mixture was heated to 91-97°C for one hour at pH of 9.3.with mixing at about 2700 rpm.

The mixture was filtered, washed and dried as described in Example 1 and the particles were ground and sieved through a 100 mesh screen and dried again.
Measured SiO₂: 13.0%

### Example 8

In this Example, no citric acid was used. The aqueous mixture of titanium dioxide was prepared, stirred then sonicated as described in Example 2. The initial pH was in the range of 3.4-3.8. The mixture was heated to 92°C. The pH was adjusted to 9.2 using 1.1 g 10% NaOH. The pH of the mixture was maintained in a range of 9-9.5 using HCI (38%, 39.20 g, 50% dilute) while adding 55.56 g sodium silicate solution (27 wt.% SiO₂) drop wise over about 27 minutes. The mixture was heated to 94°C for one hour at pH of 9.4.with mixing at about 3500 rpm.

The mixture was filtered, washed and dried as described in Example 1 and the particles were ground and sieved through a 100 mesh screen and dried again.
Measured SiO₂: 20.0%

### Example 9

In this Example, no citric acid was used. The aqueous mixture of titanium dioxide was prepared, stirred then sonicated as described in Example 2. The initial pH was in the range of 3.0-3.1. The mixture was heated to 92°C. The pH was adjusted to 9.1-9.5 using about 1.6 g 10% NaOH and maintained at that pH. The mixture was heated to 90-98°C for one hour at pH of 9.5. The mixture was filtered, washed and dried as described in Example 1 but it was noted that filtering and washing was slower than Examples made with sodium silicate. The dried material had a tan color. The particles were ground and sieved through a 100 mesh screen and dried again.
Measured SiO₂ = 0%
Measured Al₂O₃ = 0%

### Example 10

In this Example, no citric acid was used. The aqueous mixture of titanium dioxide was prepared, stirred then sonicated and pH adjusted as in Example 1. The mixture was heated to 60°C.

Then 27.0g sodium aluminate (27.8 wt% alumina) was added while keeping the pH in the range of 6-8 using 19.5 g of concentrated (38%) HCI. The mixture was then stirred for 20 minutes maintaining the pH and temperature.

The material was then filtered, washed, dried, and crushed as in Example 1.
Measured Al₂O₃ = 4.7%

**Table 1**

| Example | % SiO₂ | % Al₂O₃ | Delta b*¹ | PSD² |
|---|---|---|---|---|
| 1 | 3.9 | 5.7 | 1.7 | 54 |
| 2 | 4.4 | 3.2 | 3.5 | 50 |
| 3 | 4.1 | 4.4 | 5.4 | 65 |
| 4 | 10.1 | 14.5 | 1.0 | 61 |
| 5 | 0 | 0 | 27 | 13 |
| 6 | 8.3 | 0 | 17.6 | 45 |
| 7 | 13.0 | 0 | 12.6 | 61 |
| 8 | 20.0 | 0 | 4.3 | 85 |
| 9 | 0 | 0 | 25 | 32 |
| 10 | 0 | 4.7 | 23 | - |

| | | | | |
|---|---|---|---|---|
| ¹ As determined by the Vitamin C Yellowing Test ² As determined by the MICROTRAC UPA | | | | |

The delta b* (an indication of chemical activity) values of Examples 6, 7 and 8 show that increasing the % silica lowers the delta b* values which indicates that higher levels of silica will lead to a more chemically stable product. However, as the silica content increases the particles have a greater tendency to form agglomerates, as indicated by the PSD values. Example 2 shows that titanium dioxide particles having silica and alumina coatings in accordance with this invention have a low delta b* value indicating good chemical stability especially in comparison to untreated material (Example 5) and, in addition, the agglomeration is substantially reduced, as indicated by the PSD values. Thus, silica and alumina coated titanium dioxide nanoparticles made in accordance with this invention having low surface treatment levels have chemical stability properties which are as good as, if not better than, titanium dioxide particles that contain high silica levels. Examples 1, 2, and 3 show that the treatment of this invention can also be used with titanium dioxide nanoparticles formed by different processes with good effectiveness and produce chemically stable particles, especially compared to the untreated material (Example 5), that have reduced agglomeration compared to silica only treated particles (Example 8).

### Example 11

This example demonstrates the use of phosphoric acid as the densifying agent.

The following materials were added to a 1000 ml plastic beaker, in order: 50.00 g Degussa P25 titanium dioxide in 400 ml total volume using deionized polished water. The mixture was stirred then sonicated for 3 minutes at a power of 7. The mixture was then poured into a 600 ml stainless steel beaker equipped with an electric stirrer (Dispermat), temperature probe and pH probe. The mixture was agitated using a propeller blade. The initial pH of the mixture was 3.5. The mixture was neutralized to pH of 7 using 0.6 g sodium hydroxide. The mixture was heated to about 40-42°C. A pH of 7 was maintained while adding 5.13g phosphoric acid (85 wt.%) and 16.7 g sodium aluminate solution until the phosphoric acid was used. The pH was maintained at 7 while simultaneously adding 10 g sodium aluminate and 20.9 g hydrochloric acid until the remaining sodium aluminate was used. The mixture was stirred for 30 minutes.

The mixture was filtered and washed with deionized polished water to less than 143 mhos/cm conductance using 3600 g water and 113 micro mhos/cm.

The product was vacuum dried for 30 minutes the enough ethanol was added to cover the cake. The ethanol treated cake was held for 15 minutes then vacuum dried for about 30 minutes. The cake was put into an aluminum tray and dried in a vacuum oven at 125°C overnight, ground and sieved through a 35 mesh screen and dried again.

## Claims

1. A process comprising
(a) forming a slurry of titanium dioxide nanoparticles;
(b) contacting the scurry of titanium dioxide nanoparticles with a densifying agent;
(c) contacting the slurry with a source of metal oxide selected from the group consisting of a source of alumina, a source of silica or both; and
(d) recovering the treated titanium dioxide nanoparticles formed in step (c).

2. The process of claim 1 wherein in step (c) the slurry is contacted with a source of alumina under conditions sufficient to deposit alumina onto the nanoparticles in an amount ranging from 5 weight percent to 15 weight percent based on the weight of the titanium dioxide nanoparticles in the mixture.

3. The process of claim 1 or claim 2 wherein in step (c) the slurry is contacted with the source of silica under conditions sufficient to deposit silica onto the nanoparticles in an amount ranging from 5 weight percent to 18 weight percent based on the weight of the titanium dioxide nanoparticles in the mixture.

4. The process of any one of the preceding claims further comprising contacting the slurry of titanium dioxide nanoparticles with sodium aluminate prior to contacting the slurry with the densifying agent.

5. The process of any one of the preceding claims in which the source of silica is sodium silicate and the pH of the slurry is at least 10 when the slurry is contacted with the sodium silicate.

6. The process of any one of the preceding claims in which the source of alumina is sodium aluminate and the pH of the slurry ranges from 5 to 9 when the slurry is contacted with the sodium aluminate.

7. The process of any one of the preceding claims in which the densifying agent is added to the slurry to a concentration of from 0.1 to 3% based on the weight of the titanium dioxide nanoparticles.

8. The process of any one of the preceding claims in which the slurry is contacted with a source of alumina and a source of silica in step (c).

9. The process of claim 8 wherein in step (c) the slurry is first contacted with a source of silica and subsequently contacted with a source of alumina.

10. The process of any one of the preceding claims further comprising contacting the treated titanium dioxide particles with an organic composition.

11. The process of claim 10 in which the organic composition comprises at least one of octyltriethoxysilane, aminopropyltriethoxysilane, polyhydroxystearic acid, and polyhydroxy siloxide.

12. The process of any one of the preceding claims in which the treated titanium dioxide particles are silanized.

13. The process of any one of the preceding claims in which the densifying agent is citric acid.

14. The process of any one of claims 1 to 12 in which the densifying agent is a source of phosphate.

15. The process of any one of claims 1 to 12 in which the densifying agent is a sulfate ion.

16. Titanium dioxide nanoparticles obtainable by the process as defined in any one of the preceding claims.

17. A composition for screening ultra violet radiation comprising titanium dioxide nanoparticles as defined in claim 16 dispersed in an organic or aqueous medium.

18. A thermoplastic composition comprising titanium dioxide nanoparticles as defined in claim 16 dispersed in a thermoplastic material.

## Patentansprüche

1. Verfahren umfassend
a) das Bilden einer Aufschlämmung von Titandioxid-Nanopartikeln;
b) das Kontaktieren der Aufschlämmung von Titandioxid-Nanopartikeln mit einem Verdichtungsmittel;
c) das Kontaktieren der Aufschlämmung mit einer Quelle von Metalloxid ausgewählt aus der Gruppe bestehend aus einer Quelle von Aluminiumoxid, einer Quelle von Siliciumdioxid oder beiden; und
d) das Gewinnen der behandelten Titandioxid-Nanopartikel, die in Schritt (c) gebildet worden sind.

2. Verfahren nach Anspruch 1, wobei in Schritt (c) die Auschlämmung mit einer Quelle von Aluminiumoxid unter Bedingungen kontaktiert wird, die ausreichen, um Aluminiumoxid auf die Nanopartikel in einer Menge im Bereich von 5 Gewichtsprozent bis 15 Gewichtsprozent, auf das Gewicht der Titandioxid-Nanopartikel in der Mischung bezogen, abzusetzen.

3. Verfahren nach Anspruch 1 oder Anspruch 2, wobei in Schritt (c) die Auschlämmung mit der Quelle von Siliciumdioxid unter Bedingungen kontaktiert wird, die ausreichen, um Siliciumdioxid auf die Nanopartikel in einer Menge im Bereich von 5 Gewichtsprozent bis 18 Gewichtsprozent, auf das Gewicht der Titandioxid-Nanopartikel in der Mischung bezogen, abzusetzen.

4. Verfahren nach einem der vorhergehenden Ansprüche, des Weiteren umfassend das Kontaktieren der Aufschlämmung von Titandioxid-Nanopartikeln mit Natriumaluminat vor dem Kontaktieren der Aufschlämmung mit dem Verdichtungsmittel.

5. Verfahren nach einem der vorhergehenden Ansprüche, wobei die Quelle von Siliciumdioxid Natriumsilicat ist und der pH-Wert der Aufschlämmung mindestens 10 beträgt, wenn die Aufschlämmung mit dem Natriumsilicat kontaktiert wird.

6. Verfahren nach einem der vorhergehenden Ansprüche, wobei die Quelle von Aluminiumoxid Natriumaluminat ist und der pH-Wert der Aufschlämmung im Bereich von 5 bis 9 liegt, wenn die Aufschlämmung mit dem Natriumaluminat kontaktiert wird.

7. Verfahren nach einem der vorhergehenden Ansprüche, wobei das Verdichtungsmittel der Aufschlämmung bis zu einer Konzentration von 0,1 bis 3 Prozent, auf das Gewicht der Titandioxid-Nanopartikel bezogen, zugegeben wird.

8. Verfahren nach einem der vorhergehenden Ansprüche, wobei die Aufschlämmung in Schritt (c) mit einer Quelle von Aluminiumoxid und einer Quelle von Siliciumdioxid kontaktiert wird.

9. Verfahren nach Anspruch 8, wobei in Schritt (c) die Aufschlämmung zuerst mit einer Quelle von Siliciumdioxid und daraufhin mit einer Quelle von Aluminiumoxid kontaktiert wird.

10. Verfahren nach einem der vorhergehenden Ansprüche, des Weiteren umfassend das Kontaktieren der behandelten Titandioxid-Nanopartikel mit einer organischen Zusammensetzung.

11. Verfahren nach Anspruch 10, wobei die organische Zusammensetzung mindestens eines von Octyltriethoxysilat, Aminopropyltriethoxysilat, Polyhydroxystearinsäure und Polyhydroxysiloxid umfasst.

12. Verfahren nach einem der vorhergehenden Ansprüche, wobei die behandelten Titandioxid-Nanopartikel silanisiert werden.

13. Verfahren nach einem der vorhergehenden Ansprüche, wobei das Verdichtungsmittel Zitronensäure ist.

14. Verfahren nach einem der Ansprüche 1 bis 12, wobei das Verdichtungsmittel eine Quelle von Phosphat ist.

15. Verfahren nach einem der Ansprüche 1 bis 12, wobei das Verdichtungsmittel ein Sulfation ist.

16. Titandioxid-Nanopartikel erhältlich durch das Verfahren wie in einem der vorhergehenden Ansprüche definiert.

17. Zusammensetzung zum Screenen von Ultraviolettstrahlung umfassend Titandioxid-Nanopartikel wie in Anspruch 16 definiert, die in einem organischen oder wässrigen Medium dispergiert sind.

18. Thermoplastische Zusammensetzung umfassend Titandioxid-Nanopartikel, wie in Anspruch 16 definiert, die in einem thermoplastischen Material dispergiert sind.

## Revendications

1. Procédé comprenant
(a) la formation d'une suspension de nanoparticules de dioxyde de titane;
(b) la mise en contact de la suspension de nanoparticules de dioxyde de titane avec un agent densifiant;
(c) la mise en contact de la suspension avec une source d'oxyde métallique choisie dans le groupe constitué par une source d'alumine, une source de silice ou les deux; et
(d) la récupération des nanoparticules de dioxyde de titane traitées formées dans l'étape (c).

2. Procédé selon la revendication 1, dans lequel, dans l'étape (c), la suspension est mise en contact avec une source d'alumine dans des conditions suffisantes pour déposer l'alumine sur les nanoparticules en une quantité allant de 5 pour cent en poids à 15 pour cent en poids par rapport au poids des nanoparticules de dioxyde de titane dans le mélange.

3. Procédé selon la revendication 1 ou la revendication 2, dans lequel, dans l'étape (c), la suspension est mise en contact avec la source de silice dans des conditions suffisantes pour déposer la silice sur les nanoparticules en une quantité allant de 5 pour cent en poids à 18 pour cent en poids par rapport au poids des nanoparticules de dioxyde de titane dans le mélange.

4. Procédé selon l'une quelconque des revendications précédentes, comprenant en outre la mise en contact de la suspension de nanoparticules de dioxyde de titane avec de l'aluminate de sodium avant la mise en contact de la suspension avec l'agent densifiant.

5. Procédé selon l'une quelconque des revendications précédentes, dans lequel la source de silice est le silicate de sodium et le pH de la suspension est d'au moins 10 lorsque la suspension est mise en contact avec le silicate de sodium.

6. Procédé selon l'une quelconque des revendications précédentes, dans lequel la source d'alumine est l'aluminate de sodium et le pH de la suspension va de 5 à 9 lorsque la suspension est mise en contact avec l'aluminate de sodium.

7. Procédé selon l'une quelconque des revendications précédentes, dans lequel l'agent densifiant est ajouté à la suspension à une concentration de 0,1 à 3% par rapport au poids des nanoparticules de dioxyde de titane.

8. Procédé selon l'une quelconque des revendications précédentes, dans lequel la suspension est mise en contact avec une source d'alumine et une source de silice dans l'étape (c).

9. Procédé selon la revendication 8, dans lequel, dans l'étape (c), la suspension est d'abord mise en contact avec une source de silice et ensuite mise en contact avec une source d'alumine.

10. Procédé selon l'une quelconque des revendications précédentes, comprenant en outre la mise en contact des particules de dioxyde de titane traitées avec une composition organique.

11. Procédé selon la revendication 10, dans lequel la composition organique comprend au moins l'un parmi l'octyltriéthoxysilane, l'aminopropyltriéthoxysilane, l'acide polyhydroxystéarique et le polyhydroxy siloxyde.

12. Procédé selon l'une quelconque des revendications précédentes, dans lequel les particules de dioxyde de titane traitées sont silanisées.

13. Procédé selon l'une quelconque des revendications précédentes, dans lequel l'agent densifiant est l'acide citrique.

14. Procédé selon l'une quelconque des revendications 1 à 12, dans lequel l'agent densifiant est une source de phosphate.

15. Procédé selon l'une quelconque des revendications 1 à 12, dans lequel l'agent densifiant est un ion sulfate.

16. Nanoparticules de dioxyde de titane susceptibles d'être obtenues par le procédé selon l'une quelconque des revendications précédentes.

17. Composition pour faire écran à une radiation ultraviolette, comprenant des nanoparticules de dioxyde de titane selon la revendication 16 dispersées dans un milieu organique ou aqueux.

18. Composition thermoplastique comprenant des nanoparticules de dioxyde de titane selon la revendication 16 dispersées dans un matériau thermoplastique.
